# Europäisches Patentamt

## European Patent Office

## Office européen des brevets

(19)

(11) Veröffentlichungsnummer: **0 085 342**
**B1**

(12) ## EUROPÄISCHE PATENTSCHRIFT

(45) Veröffentlichungstag der Patentschrift:
24.09.86

(21) Anmeldenummer: **83100373.6**

(22) Anmeldetag: **18.01.83**

(51) Int. Cl.⁴: **C 01 B 33/28**, B 01 J 29/06, C 07 C 5/27, C 07 C 2/86, C 07 C 1/20

(54) **Verfahren zur Herstellung von katalytisch aktiven Alumosilikaten und ihre Verwendung.**

(30) Priorität: **28.01.82 DE 3202657**

(43) Veröffentlichungstag der Anmeldung:
**10.08.83 Patentblatt 83/32**

(45) Bekanntmachung des Hinweises auf die Patenterteilung:
**24.09.86 Patentblatt 86/39**

(84) Benannte Vertragsstaaten:
**BE DE FR GB NL**

(73) Patentinhaber: **HOECHST AKTIENGESELLSCHAFT, Postfach 80 03 20, D-6230 Frankfurt am Main 80 (DE)**

(72) Erfinder: **Vogt, Wilhelm, Dr., Bellerstrasse 74, D-5030 Hürth (DE)**
Erfinder: **Glaser, Hermann, Magdalenenweg 16, D-5042 Erftstadt (DE)**
Erfinder: **Koch, Jürgen, Dr., Am Römerkanal 10a, D-5040 Brühl (DE)**
Erfinder: **Lenz, Günter, Clarenbergweg 31, D-5020 Frechen (DE)**

(56) Entgegenhaltungen:
EP-A-0 017 027
DE-A-1 814 488
DE-A-1 952 192
DE-B-1 197 855
DE-B-1 203 239
US-A-3 244 766
US-A-4 211 756

CHEMICAL ABSTRACTS, Band 73, Nr. 8, 24. August 1970, Seite 94, Nr. 37011n, Columbus, Ohio, USA; C. COLEILA: "Zeolitization of sodium oxide-alumina-silica system II. Glass with a fixed silica-alumina ratio and variable soda-alumina ratios"
CHEMICAL ABSTRACTS, Band 73, Nr. 8, 24. August 1970, Seite 94, Nr. 32013q, Columbus, Ohio, USA; R. AIELLO et al.: "Zeolitization of sodium oxide-alumina-silica glass. III. Mechanism of the glass-to-zeolite conversion"
CHEMICAL ABSTRACTS, Band 80, Nr. 10, 11. März 1974, Seite 106, Nr. 49797f, Columbus, Ohio, USA; R. AIELLO et al.: "Glass zeolitization of the sodium

(56) Entgegenhaltungen: (Fortsetzung)
oxide-aluminum oxide-silicon dioxide system. IV. Synthesis and property of a new sodium zeolite"
CHEMICAL ABSTRACTS, Band 90, Nr. 10, 5. März 1979, Seite 152, Nr. 74544k, Columbus, Ohio, USA; D.B. HAWKINS et al.: "Hydrothermal synthesis of clinoptilolite and comments on the assemblage phillipsite-clinoptilolite-mordenite"
CHEMICAL ABSTRACTS, Band 84, Nr. 26, 28. Juni 1976, Seite 106, Nr. 182107e, Columbus, Ohio, USA; R. AIELLO et al.: "Production of large-port zeolites"
DERWENT JAPANESE PATENTS REPORT, Band U, Nr. 14, veröffentlicht am 8. Mai 1973, Seite 3, Nr. 74765R, Derwent Publications, London, GB;
CHEMICAL ABSTRACTS, Band 75, Nr. 10, 6. September 1971, Seite 285, Nr. 67863a, Columbus, Ohio, USA; R. AIELLO et al.: "Zeolite formation from synthetic and natural glasses"
CHEMICAL ABSTRACTS, Band 64, Nr. 13, 20. Juni 1966, Spalte 18538b, Columbus, Ohio, USA; R. SERSALE et al.: "Synthesis and thermal behavior of phillipsite"

EP 0 085 342 B1

## Beschreibung

Die vorliegende Erfindung betrifft ein Verfahren zur Herstellung von katalytisch aktiven Alumosilikaten aus Pulvern von Borosilikatgläsern durch hydrothermale Kristallisation, wobei man die hydrothermale Kristallisation während 10 bis 500 Stunden in Gegenwart einer alkalische Reaktion aufweisenden wäßrigen Lösung mindestens einer anorganischen Alkaliverbindung durchführt und ihre Verwendung zum Dehydratisieren von sauerstoffhaltigen organischen Verbindungen unter Bildung von Kohlenwasserstoffen sowie zur Isomerisierung bzw. Alkylierung von Kohlenwasserstoffen.

Aus der EP-A-0 017 027 ist ein Verfahren zur Herstellung eines katalytisch aktiven Zeolithen durch hydrothermale Kristallisation aus Glas in Gegenwart von Aminen wie Hexamethylendiamin bekannt, wobei der Feststoffgehalt im Reaktionsgemisch größer als 25 Gewichts% ist. Die so hergestellten Zeolithe sind als Katalysatoren zur Umsetzung von niedrigen Alkoholen oder Dialkyläthern zu aromatischen Kohlenwasserstoffen oder Olefinen geeignet.

Beim Verfahren zur Herstellung von als Katalysatoren oder Trägern geeigneten Alumosilikaten nach der EP-A-0 002 900 wird eine Mischung von Silikaten, Aluminiumoxid, einer Alkaliverbindung und Wasser bei Temperaturen von 80 bis 210°C und Drucken von etwa 5 bis 28 bar während einer Reaktionszeit von weniger als 4 Stunden in Gegenwart eines oder mehrerer substituierter sekundärer oder tertiärer Amine oder unsubstituierter tertiärer Amine umgesetzt.

Bei den beiden genannten Verfahren ist es erforderlich, erhebliche Mengen von Aminen auf das Glas einwirken zu lassen, um die Bildung von Alumosilikaten zu erreichen, wodurch deren Herstellung verteuert und die Beseitigung der Abfallstoffe problematischer wird.

Aus der US-A-4 211 758 ist ein Verfahren zur Herstellung von kristallinen Alumosilikaten vom Zeolith S-Typ durch Behandeln von pulverisiertem PYREX®-Glas mit einer wäßrigen Lösung von $Na_2CO_3$ und $NaHCO_3$ bei Temperaturen von 80 bis 120°C, insbesondere beim Siedepunkt unter Normaldruck von ungefähr 102°C, bekannt, wobei die Reaktionszeit 24 bis 168 Stunden beträgt.

Beim Verfahren zur Herstellung von Zeolithen nach der DE-A-1 814 488 wird zerkleinertes Borosilikatglas in verdünnte Natronlauge eingetragen und unter Rühren 12 bis 48 Stunden auf 60 bis 100°C erhitzt.

Aus der US-A-3 244 766 ist ein Verfahren zur Entwässerung von Alkoholen zur Gewinnung von Olefinen bekannt, wobei Alkoholdampf bei Temperaturen von unter 300°C mit einem Katalysator in Kontakt gebracht wird, welcher aus Mordenit in der H-Form besteht. Dieser Mordenit wurde gemäß der DE-B-1 197 855 aus Bimstein und Natriumsilikat-Lösung im Autoklav bei Temperaturen bis 190°C hergestellt.

In der GB-A-1 355 346 ist die Isomerisierung von Alkylbenzolen in Gegenwart eines Katalysators beschrieben, welcher aus einem Träger aus Siliciumdioxid oder aus Siliciumdioxid/Aluminiumoxid oder aus Alumosilikat, beispielsweise einem Zeolithen, besteht, wobei auf der Oberfläche des Trägers eine Schicht aus Aluminiumoxid aufgebracht ist.

Überraschenderweise wurde gefunden, daß sich durch hydrothermale Kristallisation von Borosilikatglas-Pulvern auch in Abwesenheit von Aminen und unter Verzicht auf Alkalicarbonate, Alkalihydroxide oder Wasserglas als Ätzmittel katalytisch aktive Alumosilikate bilden. Dazu werden erfindungsgemäß die Pulver von Borosilikatgläsern mit einer Korngröße kleiner als 1 mm mit der 1 bis 40 Gewicht% Alkaliphosphat enthaltenden wäßrigen Lösung bei Temperaturen von 120 bis 200°C in einem Autoklaven unter ständigem Rühren behandelt.

Das Verfahren gemäß der Erfindung kann noch dadurch ausgestaltet sein, daß das mit Wasser ausgewaschene Produkt zur Alkalientfernung mit einer Ammoniumnitratlösung gekocht wird und daß der abfiltrierte Rückstand bei Temperaturen von 60 bis 110°C getrocknet und schließlich geglüht wird.

Schließlich können die erfindungsgemäß hergestellten Alumosilikate zum Dehydratisieren von Alkoholen, Äthern und anderen sauerstoffhaltigen organischen Verbindungen unter Bildung von Kohlenwasserstoffen oder zur Isomerisierung und/oder Alkylierung von Kohlenwasserstoffen verwendet werden.

Die nach dem erfindungsgemäßen Verfahren hergestellten Alumosilikate liegen in der Na-Form vor. Wird für ihren katalytischen Einsatz die H-Form gewünscht, so läßt sich durch Auswaschen mit dem Ammoniumsalz einer Mineralsäure zunächst die $NH_4$-Form erhalten, welche durch Tempern bei 300 bis 600°C in die H-Form überführbar ist. Weiterhin kann die M-Form der Alumosilikate in üblicher Weise auch durch Behandeln der Na-Form mit Mineralsäuren erhalten werden.

Die nach dem Verfahren gemäß der Erfindung hergestellten Alumosilikate bestehen als Ergebnis der mikroskopischen Untersuchung aus einem Hauptanteil von etwa der Korngröße des eingesetzten Glases sowie aus einem kleineren Feinanteil von einigen μm Korngröße, welcher ursprünglich nicht enthalten war. Beide Anteile zeigen beispielsweise nach Umwandlung in die H-Form katalytische Aktivität, so daß zumindest ein Teil der katalytischen Aktivität von der Oberfläche der Körner ausgeht, welche vor der hydrothermalen Behandlung Glas waren. Durch Verwendung eines entsprechend geformten Ausgangsmaterials sind daher erfindungsgemäß hergestellte Alumosilikate direkt als Festbett- oder Wirbelbettkatalysatoren verwendbar.

Festbett- und Wirbelbettkatalysatoren können auch erhalten werden, indem man die Alumosilikate gemäß der Erfindung mit verfestigenden Materialien, beispielsweise

Tonsil®, vermischt und zu Formkörpern verbindet.

Die erfindungsgemäßen Alumosilikate weisen in ihrem Röntgendiagramm im Gegensatz zu bekannten synthetisch hergestellten Alumosilikaten in markanter Weisen im Bereich von 12,5 bis 13,5.10⁻¹⁰ m eine breite Bande starker Intensität auf.

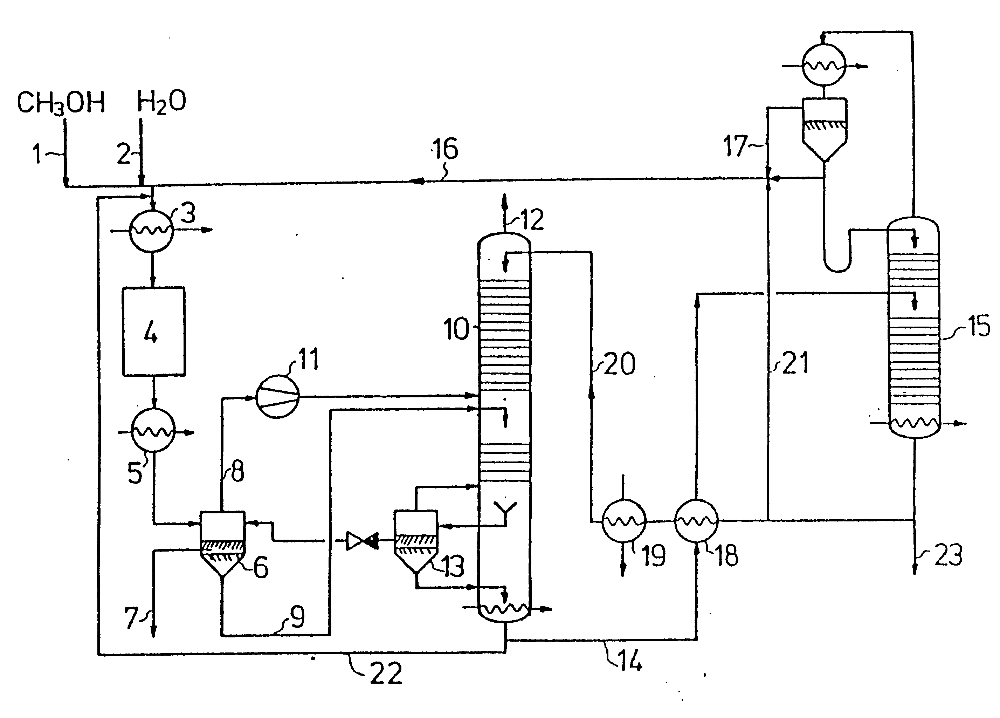

Der in folgenden Beispiel aus dem erfindungsgemäßen Alumosilikat hergestellte Katalysator wurde in einer Anlage zur Gewinnung von Kohlenwasserstoffen durch Spaltung von Methanol in Anwesenheit von Wasser eingesetzt, wie sie in der beigefügten Figur dargestellt ist:

Über die Eingangsleitungen (1, 2) werden Methanol und Wasser im Volumenverhältnis 1:1 in den Verdampfer 3 gepumpt. Das verdampfte Methanol-Wasser-Gemisch wird mit einer Temperatur von 320°C und bei einem Druck von 1,2 bar in den Festbettreaktor 4 geleitet, in welchem sich der Katalysator in Form von Extrudaten befindet. Das den Festbettreaktor 4 verlassende Gasgemisch wird im Kondensator 5 auf etwa 25°C abgekühlt und im Abscheider 6 in eine Öl-, eine Wasser- und eine Gasphase zerlegt. Die aus höheren Aliphaten und Aromaten bestehende Ölphase wird über die Leitung 7 aus der Anlage abgezogen. Die wäßrige Phase wird über die Leitung 9 auf den Abtriebsteil der Waschkolonne 10 gepumpt, während die Gasphase über die Leitung 8 und den Kompressor 11 der Waschkolonne 70 unterhalb des Absorptionsteils mit 20 bar zugeführt wird. Der Kopf der Waschkolonne 10 wird über die Leitung 20 mit Wasser von 25°C beaufschlagt, um das im Festbettreaktor 4 nicht umgesetzte Methanol sowie den als Zwischenprodukt gebildeten Dimethylether aus dem Gasgemisch auszuwaschen. Über die Leitung 12 ist aus der Waschkolonne ein im wesentlichen aus Kohlenwasserstoffen bestehendes Gasgemisch entnehmbar.

Das beim Durchgang durch den Festbettreaktor 4 nicht umgesetzte Methanol sowie der entstandene Dimethylether werden zusammen mit dem Waschwasser vom Fuß der Waschkolonne 10 über die Leitung 14 der Abstreifkolonne 15 zugeführt. Vom Kopf der Abstreifkolonne 15 werden durch die Leitung 16 Methanol und gasförmiger Dimethylether (über die Leitung 17) über den Verdampfer 3 in den Festbettreaktor 4 zurückgeleitet.

## Beispiel 1

In einem 5,1-Edelstahlautoklaven werden 500 g Duran®-Glaspulver einer Korngröße von weniger als 300 μ und 2 l einer 38 %igen Lösung von Trinatriumphosphat-Dodecahydrat (Na₃PO₄. 12 H₂O) eingefüllt. Der Autoklav wird unter ständigem Rühren 6 Tage auf 175°C gehalten. Das nach Abkühlen aus dem Autoklaven entnommene feinkörnige Produkt wird mit Wasser gewaschen und anschließend zur Alkalientfernung dreimal mit 10 %iger Ammoniumnitratlösung gekocht. Der abfiltrierte Rückstand wird bei 60 bis 170°C getrocknet und schließlich 2 Stunden bei 500°C geglüht. Das nunmehr in der H-Form vorliegende Alumosilikat wird mit Tonsil® als Bindemittel im Gewichtsverhältnis 4:1 vermischt und einem Walzenextruder zugeführt, mit Hilfe dessen ein Extrudat von 3 mm Durchmesser hergestellt wird.

Das Extrudat wird in den Festbettreaktor 4 der oben beschriebenen Anlage eingesetzt, wobei die Katalysatorleistung 60 g Ethylen neben 100 g Propylen und 26 g Butylen pro Liter Katalysator x Stunde beträgt.

## Patentansprüche

1) Verfahren zur Herstellung von katalytisch aktiven Alumosilikaten aus Pulvern von Borosilikatgläsern durch hydrothermale Kristallisation, wobei man die hydrothermale Kristallisation während 10 bis 500 Stunden in Gegenwart einer alkalische Reaktion aufweisenden wäßrigen Lösung mindestens einer anorganischen Alkaliverbindung durchführt, dadurch gekennzeichnet, daß man die Pulver von Borosilikatgläsern mit einer Korngröße kleiner als 1 mm mit der 1 bis 40 Gewichts% Alkaliphosphat enthaltenden wäßrigen Lösung bei Temperaturen von 120 bis 200°C in einem Autoklaven unter ständigem Rühren behandelt.

2) Verfahren nach Anspruch 1, dadurch gekennzeichnet, daß das mit Wasser ausgewaschene Produkt zur Alkalientfernung mit einer Ammoniumnitratlosung gekocht wird und daß der abfiltrierte Rückstand bei Temperaturen von 60 bis 110°C getrocknet und schließlich geglüht wird.

3) Verwendung der nach dem Verfahren gemäß den Ansprüchen 1 oder 2 hergestellten Alumosilikate zum Dehydratisieren von Alkoholen, Äthern und anderen sauerstoffhaltigen organischen Verbindungen unter Bildung von Kohlenwasserstoffen.

4) Verwendung der nach dem Verfahren gemäß den Ansprüchen 1 oder 2 hergestellten Alumosilikate zur Isomerisierung und/oder Alkylierung von Kohlenwasserstoffen.

## Claims

1. Process for making catalytically active aluminosilicates from powders of borosilicate glasses by hydrothermal crystallization, wherein the hydrothermal crystallization is effected over a period of 10 - 500 hours in the presence of an aqueous solution producing an alkaline reaction of at least one inorganic alkali metal compound, which comprises treating, with continuous agitation, the borosilicate glass powders having a particle size of less than 1 mm with the aqueous solution containing 1 - 40 wgt % alkali metal phosphate at temperatures of 120 200° C in an autoclave.

2. Process as claimed in claim 1, wherein, in order to remove the alkali metal, the water-washed product is boiled with an ammonium nitrate solution and the filtered residue is dried at temperatures of 80 - 110° C and ultimately calcined.

3. Use of the aluminosilicates made by the process as claimed in claim 1 or 2 for dehydrating alcohols, ethers and further oxygen-containing organic compounds with formation of hydrocarbons.

4. Use of the aluminosilicates made by the process as claimed in claim 1 or 2 for isomerizing and/or alkylating hydrocarbons.

## Revendications

1. Procédé de préparation d'aluminosilicates catalytiquement actifs à partir de poudres de verres de borosilicates par cristallisation hydrothermique, dans lequel on effectue la cristallisation hydrothermique pendant 10-500 heures en présence d'une solution aqueuse à réaction alcaline d'au moins un composé alcalin minéral, caractérisé en ce que l'on traite avec agitation constante les poudres de verres de borosilicates d'une granulométrie inférieure à 1 mm par la solution aqueuse à 1-40 % en poids de phosphate alcalin à des températures de 120-200° C dans un autoclave.

2. Procédé selon la revendication 1, caractérisé en ce que, pour éliminer l'alcali, on fait bouillir le produit lave à l'eau avec une solution de nitrate d'ammonium, on sèche à des températures de 60-110° C le résidu filtré et enfin on le calcine.

3. Utilisation des aluminosilicates préparés par le procédé selon la revendication 1 ou 2 pour la déshydratation d'alcools, d'éthers et d'autres composes organiques contenant de l'oxygène avec formation d'hydrocarbures.

4. Utilisation des aluminosilicates préparés par le procédé selon la revendication 1 ou 2 pour l'isomérisation et/ou l'alkylation d'hydrocarbures.